# EUROPEAN PATENT APPLICATION

(11) **EP 4 512 466 A1**
(43) Date of publication of application: **26.02.2025**
(21) Application number: 23193535.4
(22) Date of filing: 25.08.2023
(51) Int. Cl.: A61N 5/06

(54) **LOW POWER LASER THERAPY DEVICE WITH UNIFORM INTENSITY DISTRIBUTION**

(71) Applicant: LYMA Life Ltd., London NW1 5QT (GB)
(72) Inventor: GOFF, Simon, London (GB); RÓZSA, Tamás, Mogyoród (HU)
(74) Representative: CMS Cameron McKenna Nabarro Olswang LLP

(57) **Abstract**

Low power laser therapy device, which comprises at least two optical systems (20, 30) each of them comprising a laser light source (10a or 10b), a beam expander (11 or 21) in front of said light sources, a raster arrangement of first light scattering elements (12) on the frontal surface of the associated beam expander, and the device comprise a housing (31, 32) around the optical systems (10, 30) having a mouth opening covered by a closing member, wherein in front of the optical systems (20, 30, 40 or 50) a second light scattering element (13) is arranged that fills said mouth opening and comprising a raster arrangement of pyramids arranged in rows and columns, and the first light scattering elements (11) are made as pyramids, and the beam expanders (11, 21) and the first and second light scattering elements (12, 13) are made from transparent material that does not affect the polarization of light rays passing therethrough.

## Description

### Background

The invention relates to a low power laser therapy device, which comprises at least two optical systems each of them comprising a laser light source, a beam expander in front of said light source and a raster arrangement of first light scattering elements on the frontal surface of the associated beam expander. The device additionally comprises a housing around the optical systems that has a mouth opening covered by a closing member.

Several laser therapy devices are known which differ from each other on features such as size, type of preferred treatment and whether they have a power supply from the mains or whether the power source is independent from the mains, such as a battery power source.

The present invention relates to such independent types of devices, operating by battery power. In the case of devices connected to the mains, special precautions should be made to prevent patients from any possible electrical shock, and such precautions are often implemented by circuits which increase the size and the cost of the devices. Moreover, connecting wires in these circuits often limit the free movement and use.

WO 2017/001876 describes a light therapy device, which uses a beam expander in front of the light source. On a frontal surface of the beam-expander, a raster of small diverting lenses is arranged. In a small distance in front of the beam expander with the diverting lenses thereon, a flat beam expander is arranged made of white, porous polyethylene with a high degree of light attenuation. The reason of using such a light scattering element lies in that the device is intended for the treatment of the human eye being very sensitive to high light intensities and a high attenuation is needed.

This device comprises a long body in which the battery can be arranged and a head portion that provides space for the optical elements.

In EP 3 787 742 B1 a portable, battery-operated laser therapy device is described, in which the available power emitted by the laser light sources is not attenuated and the optical elements are arranged in such a way that their intensities are added in a central zone to increase the penetration depth of the infrared laser light in human tissues. The device uses at least three laser sources with a beam expander and beam scattering system placed in front of the light sources. The device is provided with cooling ribs to dissipate the substantial amount of heat emitted by the plurality of light sources.

Laser therapy may be used at skin depths other than the deep tissues, for example, in the epidermis, dermis or fat layers. In laser therapy, it is advantageous to irradiate large areas of skin with laser light, with the treated area being as large as possible within the power and size constraints of a battery-operated portable device. Irradiating the surface of skin under the device in a uniform way has many benefits, especially for cosmetic purposes.

The object of the invention is to provide a low power laser device which can meet these objectives and can generate substantially uniform intensity within the irradiated area.

### Summary of the invention

The device according to the invention is designed as defined in the attached claims.

### Brief description of the drawings

The invention will now be described in connection with preferable embodiments thereof, in which reference will be made to the attached drawings. In the drawing:
Fig. 1 is a sketch showing a light source with beam expander, first beam scattering lenses and second beam scattering lenses;
Fig. 2 shows a similar arrangement in which two lenses are arranged side-by-side having a common second scattering lens;
Fig. 3 shows the light intensity distribution of a twin light source arrangement like that shown in Fig. 2 without the second scattering lens;
Fig. 4 shows the light intensity distribution, wherein the device is similar to that shown in Fig. 3 but uses a common second light scattering lens;
Fig. 5 is a view taken along line V. - V. of Fig. 4,
Fig. 6 is a similar view to Fig. 5 but using three beam expanders and light sources
Fig. 7 is a view made in a direction opposite to line V - V of Fig. 4 and
Fig. 8 is an enlarged view of the device according to the invention.

### Detailed description

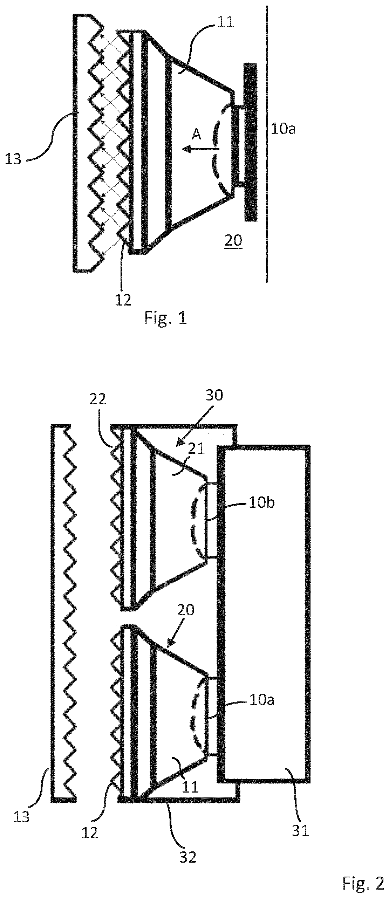

Fig. 1 is an enlarged explanatory sketch of a first optical system 20 used in the present invention in front of a single laser light source 10a. In front of the light source 10a, a beam expander 11 is arranged. The beam expander 11 is made of a light transparent material, such as polymethyl methacrylate, and has a shape designed as a widening cone in forward direction, i.e., along the main direction of light away from the light source 10a and of the axis A of the light source 10a. On the frontal surface of the beam expander 11, which is preferably a planar surface and normal to the axis A, first light scattering element 12 are provided. In. Fig. 1, the light scattering elements 12 are depicted as pyramids which are arranged immediately adjacent to each other to form a raster. At a distance in front of the frontal face of the beam expander 11, a second light scattering element 13 is arranged. In various examples, the second light scattering element 13is made of the same or similar light transparent material as the beam expander 11. However, the second light scattering element 13 need not be made of the same material as the beam expander 11 but it must be light transparent and not changing the polarization of the light passing therethrough. The second light scattering element 13 has an inner (rear) surface also designed as a raster of pyramids, facingtowards the first light scattering elements 12. In this example, the opposing (frontal) surface of the second light scattering element 12 is planar and it is also normal to the axis A.

In Fig. 1 small arrows show the path of the light rays when leaving the first light scattering element 12 and enter the surfaces of the second light scattering element 13. Owing to the design and presence of the scattering elements, there will be minimum attenuation or reflection loss between the first 12 and second 13 scattering elements. Where the scattering elements comprises a plurality of pyramids in a raster arrangement, most of the emitted light will enter normal to the surfaces of the plurality of pyramids on the second light scattering element 13 so that there will be minimum attenuation or reflection loss. To achieve an optimal saving in attenuation or reflection loss, the size of the pyramids and the distance between the two opposite surfaces of the first and second light scattering elements 12 and 13 are selected tobebetween 1,5 to 2.5 times of the height of the pyramids of the second light scattering element 13. The height of the pyramids in the second light scattering element is typically in the range of 0.8 to 1.3 mm. Pyramids in both the first 12 and second 13 light scattering elements have an apex angle between about 85° to 95 °.

Fig. 2 shows the first optical system 20 of Fig. 1 placed directly adjacent to an identical second optical system 30, which have a common second scattering element 13 being sufficiently large to cover both optical systems 20 and 30. The second optical system 30 has a second light source 10b, a beam expander 21 and first light scattering element 22. The optical system 20 and 30 may be held mechanically by a common housing 31. The housing comprises a head portion, the head comprising a cavity in which at least two optical systems may be held. As an example, the housing may comprise a wider head portion that has the hollow cavity, to surround the two or more optical systems, as described in in EP 3 787 742 B1. However, the head portion of the common housing 31 of the present invention need not be wider than a body of the housing.

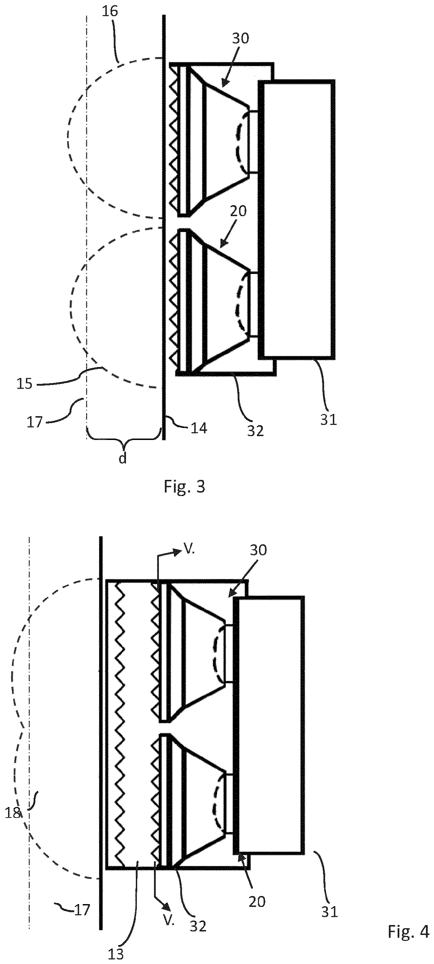

Fig. 3 is an exemplary figure showing the two optical systems 20 and 30 arranged as in Fig, 2 but where the second scattering element 13 is not used. In this exemplary figure, the second scattering element 13 is replaced by a planar, transparent closing element 14. In front of this closing element 14 two semi circles 15, 16 drawn by dashed line are shown, which illustrate the light intensity distribution curves measured in the plane of the drawing. Dot-dashed line 17 is drawn at a distance d from the rear face of the closing element 14 and is parallel thereto. The light intensity of the light sources 10a and 10b, depicted by dashed lines 15 and 16 respectively, at a distance greater than d from the rear face of the closing element 14 is only higher than line 17 in two short discrete central regions, and at all other regions it is much smaller. Optical systems with such sharply varying light distribution cannot be used for skin care, as in a large area under the line 17 the intensity is small and varies to a substantial extent.

Fig. 4 illustrates an arrangement in accordance with the present invention. The system of Fig. 4 is similar to Fig. 3, with the addition of the second scattering element 13. As an effect of the multiple reflections that take place in the second scattering element 13, a very different light intensity curve 18 is obtained, which is more uniform and intensive above the whole area of the second light scattering element 13. In a much larger region than the system depicted in Fig. 3, the intensity is higher than at the reference line 17. Moreover, the light intensity is only slightly higher than this reference intensity across the length of the second scattering element, therefore this light distribution can be considered a substantially uniform. The size of this area above the line 17 is about the same as the diameter of the front part of the housing 32. As such, the area treated by the device will be uniformly illuminated, which is a significant difference compared to the arrangement without the second light scattering element13.

In the sketches referred to herein, only the elevation view of the device has been illustrated. It will be readily understood by the person skilled in the art that the full or almost the full area behind the second light scattering element 13 and of the mouth of the frontal portion 32 of the housing can be uniformly illuminated.

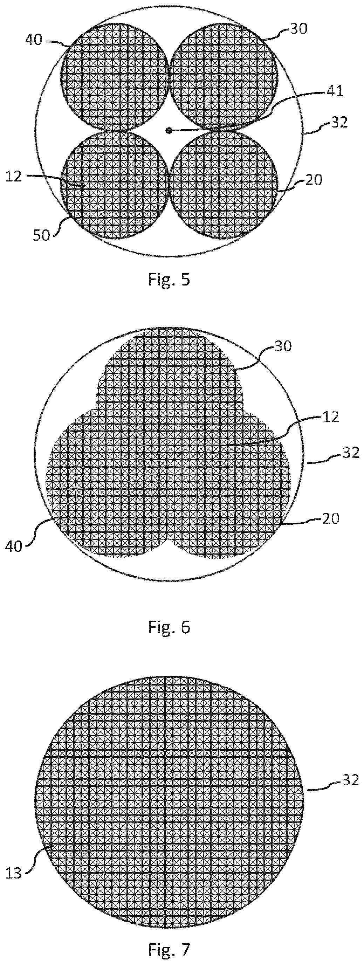

Reference is made now to Fig. 5 in which four optical systems 20, 30, 40 and 50 with circular front surfaces are arranged adjacent to each other and their center points fall in the corner points of a square and these circles contact each other. In Fig. 5, the pyramids that constitute together the first light scattering elements 12 are seen from their top surfaces. Each of the four optical systems have a respective laser light source. In examples where the respective laser light sources emit light in the infrared range of wavelengths (preferable at 808 nm or close thereto) which is not visible by the human eye, it is advantageous to place a light source 41 emitting visible light, such as a light emitting diode (LED). For example, the optical system may comprise a visible light source in a central opening among the optical systems between where the respective first light scattering elements abut. In examples where the first light scattering elements do not abut, visible light sources may be placed between adjacent first light scattering elements 12, which has the advantage of each visible light source indicating whether a respective laser light source is operating. The visible light source(s) 41 may be energized together with the laser light sources. Advantageously, the user can see when the device is turned on and is ready for treatment. The distribution shown in Fig. 4 is circularly symmetric if the four optical system are used.

A similar intensity distribution can be obtained if instead of using four optical systems only three of them is used in which their centers fall on the apex point of a regular triangle. Fig 6. shows such an arrangement. The difference compared to the embodiment of Fig. 5 lies not only in the reduced number of optical systems but also in the fact that the bodies of the three beam expanders are made from a single piece. In this example, the three cones have a united common central portion as illustrated in Fig. 6. The forming of a common body for the three optical system can be made as illustrated in Fig. 2 of the previously cited EP 3 787 742 B1. Alternatively, the three beam expanders are three distinct pieces which are shaped to fit each other and are arranged adjacent to each other to form the same shape as the single piece shown in FIG. 6. In both examples, the three optical beam expanders have a common central zone at the laser light source facing surface.

The first light scattering elements are also pyramids that are distributed in rows and columns along the whole frontal surface of the united optical systems.

Fig. 7 shows the rear surface of the second light scattering element 13 with the pyramids arranged also in rows and columns. The pyramids can be prepared as extending out from the rear surface of the plastic material of the element 13 which is at the same time the frontal closing member of the housing 32 but can also be made as pyramid-shaped recesses or depressions made in the material of the element 13. The possibility of using inverse pyramids is also true for the first light scattering elements 12.

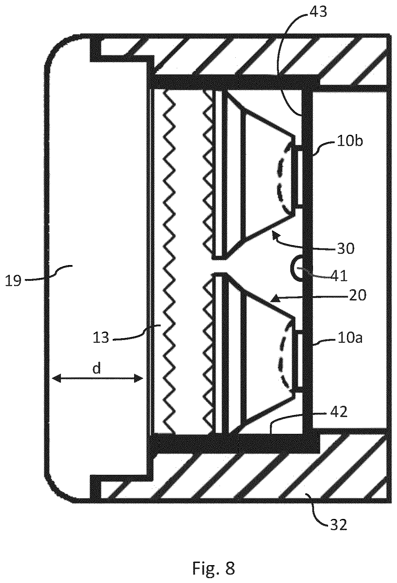

Finally, Fig. 8 shows the elevation sectional view of the preferred embodiment shown in Fig. 4 having four optical systems, in which the difference lies in that the second scattering element 13 and the transparent closing member 19 are made as one. The thickness is of the closing member in some examples, is approximately the same distance as the distance d illustrated in Fig. 4, and therefore the full-frontal surface of the device (i.e., of the closing element 19) emits laser light with substantially the same intensity. During use, the circular front surface of the closing element 19 can be slightly contacted to the skin area to be treated. The light utilization efficiency is increased if the internal surfaces of the housing 32, like the cylindrical surface 42 around and in front of the optical systems and the planar rear surface 43 are covered with a light reflecting (mirroring) layer, since the light falling on such surfaces will also be reflected and utilized.

The material of the second scattering element 13, being part of the closing element 19 and of the two beam expanders 11, 21 should not affect the polarization of the emitted laser light, and such material is e.g., the previously mentioned polymethyl methacrylate.

The size of a preferred embodiment, which is not a limiting example, can be preferably designed so that the diameter of the closing element 19 is around 40-50 mm. As another example, the distance d is around 8-12 mm. Three or four optical systems may be used and are arranged as shown in Figs. 5 and 6. The laser light sources 10 a, b, c and/or d emit light in the wavelength range which is optimum for the selected treatment where 808 nm is a preferred wavelength. The power of the individual light sources is preferably between around 100 mW and 600 mW. In case infrared light is used, which is not visible by the human eye, it is advantageous to use the pilot led light 41 which is turned on and off together with the laser lights.

The main field of use of the device is cosmetics but other kinds of uses can also be performed. As such, the cosmetic use of the low power therapy device according to any of the embodiments and examples described herein to treat skin is also provided.

The advantages of the present device include the uniform filed intensity and the high efficiency of the available light energy as well as the battery-operated use, whereby the device can be used anywhere and independent from the availability of mains supply, and there are no electrical shock hazards either.

## Claims

1. Low power laser therapy device, which comprises at least two optical systems (20, 30),wherein each optical system (20, 30) comprises a laser light source (10a or 10b), a beam expander (11 or 21) in front of said light source, a raster arrangement of first light scattering elements (12) on the frontal surface of the associated beam expander, and the device comprises a housing (31, 32) around the optical systems (10, 30) having a mouth opening covered by a closing member, **characterized in that** in front of the optical systems (20, 30, 40 or 50) a second light scattering element (13) is arranged that fills said mouth opening and comprising an arrangement of pyramids, and the first light scattering elements (12) are made as pyramids, and the beam expanders (11, 21) and the first and second light scattering elements (12, 13) are made from a non-polarizing transparent material.

2. The device as claimed in claim 1, wherein said arrangement of the first (12) and second light scattering element (13) comprises a raster arrangement of pyramids arranged in rows and columns.

3. The device as claimed in claim 1 or 2, wherein said pyramids each have quadratic base and their apex angles is between 85°and 95°.

4. The device as claimed in any of claims 1 to 3, wherein the height of said pyramids is between 0.6 and 1.3 mm.

5. The device as claimed in any of claims 1 to 4, wherein the second light scattering element (13) is integral to the closing member.

6. The device as claimed in any of claims 1 to 5, wherein the axial distance between the first and second light scattering elements (12, 13) is between 1.5 to 2.5 times of the height of the pyramids.

7. The device as claimed in any of claims 2 to 6, further comprising:
a third optical system (40),
wherein the three optical systems (20, 30, 40) have circularly symmetrical shapes and their center points fall to the apex point of a regular triangle.

8. The device as claimed in claim 7, wherein the three optical systems (20, 30, 40) beam expanders comprise a common central portion and wherein the three optical systems (20, 30, 40) comprise a common body.

9. The device as claimed in any of claims 1 to 6, further comprising:
a third optical system (40);
a fourth optical system (50),
wherein the four optical systems (20, 30, 40, 50) have circularly symmetrical shapes and their center points fall to the apex points of a square, and wherein adjacent beam expanders of the respective beam expanders of the four optical systems (20, 30, 40, 50) contact each other at respective single points.

10. The device as claimed in any of claims 1 to 9, wherein a material of the first and second light scattering elements (121 13) and/or of the beam expanders (11, 21) is polymethyl methacrylate.

11. The device as claimed in any of claims 1 to 10, wherein an inner surface (42, 43) of the housing (31, 32) comprises a light reflecting layer (42).

12. The device as claimed in any of claims 1 to 11, wherein the laser light sources (10a, 10b) are configured to emit light in an infrared range of wavelength, wherein the laser light sources (10a, 10b) are preferably configured to emit light at 808nm.

13. The device as claimed in any of claims 1to 12, which comprises at least one LED light source emitting light in the visible range of wavelength and being switched together with said laser light sources (10a, 10b).

14. The device as claimed in any of claims 1 to 14, wherein the power of each one of the laser light sources (10a, 10b) is between 100mW and 600 mW.
